# FASCICULE DE BREVET EUROPEEN

(11) **EP 3 971 092 B1**
(45) Date de publication et mention de la délivrance du brevet: **21.06.2023**
(21) Numéro de dépôt: 21306268.0
(22) Date de dépôt: 15.09.2021
(51) Int. Cl.: B65B 3/00, G16H 20/13, B65B 3/12, B65B 3/28, G01G 7/04, G01G 13/00, G01G 17/06, B65B 7/28, B65B 43/58, B65B 57/14, B65B 57/16, B65B 59/00, B65B 59/02, B65B 61/02, B65B 61/26, B65B 61/28, B67C 3/20, G07F 17/00, G07F 11/44, G07F 11/62, G07F 13/10

(54) **AUTOMATE DE CONDITIONNEMENT DE PRESCRIPTIONS ET PROCEDE ASSOCIE**
AUTOMAT ZUR VERPACKUNG VON VERSCHRIEBENEN MEDIKAMENTEN UND ENTSPRECHENDES VERFAHREN
AUTOMATON FOR PACKAGING PRESCRIPTIONS AND ASSOCIATED METHOD

(30) Priorité: 17.09.2020 FR 2009427
(43) Date de publication de la demande: 23.03.2022
(73) Titulaire: Nooddis, 01700 Beynost (FR)
(72) Inventeur: ALACOQUE, David, 01330 VILLARS-LES-DOMBES (FR); MECHERI, Tamim, 01360 BRESSOLLES (FR); BAUDELET, Cyrille, 38460 CHAMAGNIEU (FR)
(74) Mandataire: Cabinet Laurent & Charras

(56) Documents cités:
- EP-A1- 3 255 393
- FR-A1- 2 894 701
- FR-B1- 2 894 701
- US-A1- 2013 255 831

## Description

### DOMAINE TECHNIQUE

L'invention concerne un automate de conditionnement de prescriptions, c'est-à-dire une machine intégrant au moins un réservoir de médicament et délivrant des doses personnalisées du médicament en fonction de quantités prescrites. L'invention est donc associée à une base de données de prescriptions utilisée par l'automate pour délivrer les doses personnalisées.

L'invention trouve une application particulièrement avantageuse pour conditionner des médicaments, sous forme liquide, pour des patients d'un milieu hospitalier, des résidents de maisons de retraite, des résidents d'établissements pénitenciers, d'officines de pharmacie ou industrielle ou encore des élèves d'un système éducatif nécessitant une prise ponctuelle ou régulière de médicaments.

### TECHNIQUES ANTERIEURES

Pour éviter de délivrer un médicament qui n'est pas destiné à un patient, les grands hôpitaux sont classiquement dotés d'un automate de conditionnement de médicaments destiné à préparer les doses prescrites à tous les patients de l'hôpital mais également aux résidents des maisons de retraite proches et aux élèves des écoles proches nécessitant un traitement.

Un tel automate de distribution est décrit dans le document US 5 337 919 ou le document FR 2 894 701 de la présente demanderesse.

Par exemple, le document FR 2 894 701 concerne un automate de conditionnement comportant un convoyeur déplaçant des gobelets entre différents postes : un poste de remplissage du médicament prescrit, un poste de dilution du médicament conditionné, un poste de fermeture du gobelet, ainsi qu'un poste d'étiquetage du gobelet.

Ces différents postes permettent de fournir des gobelets personnalisés sur lesquels sont inscrits les noms des patients et dans lesquels sont conditionnés le médicament avec le dosage prescrit.

Pour procéder au remplissage du médicament avec la quantité prescrite, les différents médicaments disponibles dans l'automate sont agencés sur un barillet. Le barillet est entraîné en rotation jusqu'à ce que le médicament recherché soit disposé en regard du gobelet. Ensuite, la pompe associée au médicament sélectionné est activée jusqu'à ce que la quantité de médicament recherchée soit conditionnée dans le gobelet. Par exemple, pour une pompe mécanique délivrant 4 ml par poussée, l'automate calcul le nombre d'activations nécessaires de la pompe pour atteindre approximativement la quantité de médicament prescrite. Dans cet exemple, si une quantité de 20 ml est prescrite, l'automate effectue cinq pressions sur la pompe mécanique pour obtenir sensiblement 20 ml de médicament conditionné dans le gobelet.

Cependant, ce système automatisé trouve ces limites dans la précision de la quantité de médicament délivré, notamment parce que la pompe est connue pour délivrer une quantité prédéterminée de médicaments et que les quantités possibles de remplissage sont fonctions de cette quantité prédéterminée. Dans l'exemple précédent, il n'est pas possible d'obtenir un remplissage de 19 ml de médicaments avec une pompe qui, à chaque pression, délivre une quantité de 4 ml. Ainsi, une approximation est réalisée pour délivrer la quantité de médicament la plus proche possible de la quantité prescrite.

En outre, la quantité de médicament délivré peut-être faussée lorsque la pompe est partiellement obturée, par exemple lorsque des bulles sont présentes dans le conduit d'acheminement de la pompe. Ainsi, si le débit de la pompe est modifié par rapport au débit attendu, la quantité de médicament délivré peut varier sans même que l'automate en soit averti.

Le problème technique de l'invention consiste donc à fournir un automate de conditionnement de médicament avec un contrôle plus efficace de la quantité de médicament délivré. Pour répondre au problème technique de l'invention, une solution classique consisterait à utiliser une pompe mécanique avec un volume délivré par pression très faible, par exemple 0.5 ml. Cependant, le temps de remplissage serait particulièrement long avec ce type de pompe pour des quantités importantes prescrites.

### EXPOSE DE L'INVENTION

Pour résoudre le problème technique, l'invention propose d'asservir le fonctionnement de la pompe assurant le remplissage du médicament par une mesure du poids du gobelet lors du remplissage de sorte à remplir le gobelet jusqu'à ce que le poids corresponde à une quantité prescrite de médicaments.

Pour ce faire, l'invention propose d'utiliser une balance à force électromagnétique de compensation. Une balance à force électromagnétique de compensation est une balance de précision, très spécifique.

L'invention est issue d'une observation selon laquelle une balance à force électromagnétique de compensation permet de mesurer efficacement le poids de remplissage d'un médicament d'un automate de conditionnement de médicaments car elle peut être précise à la goutte près, sur une plage de valeur importante, et elle présente un temps de stabilisation rapide, tout en résistant aux contraintes de vibration et de température inhérentes à un automate de conditionnement de prescriptions.

En effet, les balances de précision classiques, c'est-à-dire les balances fonctionnant avec un principe de mesure de torsion d'une poutre, ne sont pas suffisamment précises pour ce type d'application compte tenu des disparités de volumes de remplissage mis en oeuvre.

Typiquement, un tel automate de conditionnement peut être amené à conditionner une quantité de médicament liquide comprise entre 0 et 30 mL. La masse volumique des médicaments peut varier entre 500 et 1500 kg/m³. Ainsi, le poids à mesurer lors du remplissage peut varier entre 0 et 45g, sans compter le poids du gobelet.

Or, les balances de précision permettent classiquement d'obtenir une mesure de poids avec une précision de 0.001g mais cette précision est classiquement spécifiée pour une gamme de 1 à 10g.

Contrairement à ces balances de précision classiques, une balance à force électromagnétique de compensation peut être précise à 0.001g sur une gamme de 0 à 100g.

En outre, une balance à force électromagnétique de compensation présente également une capacité de résistance aux vibrations nécessaire pour ce type d'application puisqu'un automate de conditionnement de médicaments nécessite un certain nombre de pièces mobiles pour obtenir les cadences recherchées, typiquement entre 300 et 400 conditionnements par heure, entraînant un grand nombre de vibrations qui peuvent perturber une balance de précision classique.

Selon un premier aspect, l'invention concerne un automate de conditionnement de prescriptions pour un ensemble de patients, au moyen d'une base de données comportant une quantité prescrite d'un médicament associé à chaque patient, ledit automate de conditionnement comportant :
- des gobelets destinés à être délivrés aux différents patients avec la quantité prescrite de médicament ;
- un convoyeur de déplacement des gobelets comportant des ouvertures traversantes permettant de recevoir les gobelets afin de les déplacer jusqu'à un poste de remplissage comportant :
   ▪ au moins un réservoir de médicament ;
   ▪ une tête de remplissage destinée à délivrer le médicament ;
   ▪ un pompe connectée entre le réservoir et la tête de remplissage ; et
   ▪ un moteur actionnant la pompe de sorte à délivrer le médicament contenu dans le réservoir au niveau de la tête de remplissage ; et
- un organe de supervision, connecté à la base de données, configuré pour commander la pompe de sorte à délivrer la quantité prescrite.

L'invention est caractérisée en ce que ledit poste de remplissage comporte également :
- une balance à force électromagnétique de compensation comportant un plateau ; et
- un actionneur de déplacement de ladite balance configuré pour déplacer ladite balance entre deux positions :
   ▪ une position de remplissage dans laquelle le plateau soulève un gobelet vers ladite tête de délivrance de sorte que le gobelet soulevé ne soit plus soutenu par le convoyeur et que la balance puisse mesurer l'évolution du poids du gobelet soulevé au cours du remplissage de celui-ci ; et
   ▪ une position de convoyage dans laquelle le plateau n'est pas au contact d'un gobelet de sorte que le convoyeur puisse déplacer les gobelets ;
- ledit organe de supervision étant configuré pour commander le moteur en fonction du poids mesuré par ladite balance à force électromagnétique de compensation jusqu'à ce que le poids mesuré corresponde à la quantité prescrite.

L'invention permet, ainsi, de mesurer avec précision le volume de médicament délivré dans le gobelet de sorte à garantir plus efficacement les doses de médicaments délivrées aux patients.

Pour ce faire, l'invention repose sur l'utilisation d'une balance à force électromagnétique de compensation mobile entre deux positions : une position de remplissage et une position de convoyage.

De préférence, la balance à force électromagnétique de compensation comporte :
- un levier à une première extrémité duquel le plateau est monté ;
- une cellule de détection de la déviation montée sur une seconde extrémité du levier et configurée pour détecter une déviation du levier d'une position d'équilibre ;
- un pivot placé sous le levier entre la première et la seconde extrémité de sorte que les déplacements du plateau dus à la force de gravité entraînent un déplacement d'au moins une partie de la cellule de détection de la déviation ;
- un contrepoids électromagnétique disposé sous le levier au niveau de la seconde extrémité ; et
- un régulateur, connecté à la cellule de détection de la déviation et au contrepoids électromagnétique ;
- le régulateur étant configuré pour commander, dans la position de remplissage, l'énergie électrique du contrepoids électromagnétique de sorte à s'opposer à une déviation détectée par la cellule de détection de la déviation, le poids du gobelet étant calculé en fonction de l'énergie électrique imposée au contrepoids électromagnétique pour s'opposer à une déviation dudit levier.

Ainsi, la balance à force électromagnétique de compensation utilisée dans l'invention présente une position d'équilibre qui peut être maintenue, dans la position de remplissage, en ajustant l'énergie électrique du contrepoids électromagnétique. Cette technologie spécifique permet de résister efficacement aux vibrations subies par la balance au cours du remplissage.

Selon un mode de réalisation, ladite cellule de détection de la déviation comporte :
- une source lumineuse et une photodiode, montées en regard l'une de l'autre et fixes par rapport à un carter de l'automate de conditionnement ; et
- un obturateur monté sur la seconde extrémité du levier, ledit obturateur étant muni d'une ouverture disposée entre la source lumineuse et la photodiode lorsque le levier est dans la position d'équilibre.

La source lumineuse peut être une diode électroluminescente, une diode laser ou encore un laser. Ce mode de réalisation permet d'obtenir une détection efficace d'une déviation de la position d'équilibre du levier et, ainsi, de mesurer avec précision le poids du gobelet lors du remplissage de celui-ci.

Le remplissage du gobelet peut être réalisé avec une commande linéaire. De préférence, ledit organe de supervision est configuré pour commander la pompe avec une course non linéaire comportant au moins deux vitesses de remplissage, une première vitesse jusqu'à atteindre au moins 80% de la quantité prescrite et une seconde vitesse, inférieure à la première vitesse, jusqu'à atteindre 100% de la quantité prescrite.

Pour obtenir une mesure précise du poids du gobelet lors du remplissage, la balance à force électromagnétique de compensation doit procéder à une régulation de l'énergie électrique du contrepoids électromagnétique et cette régulation peut prendre quelques millisecondes pour stabiliser le levier sur la position d'équilibre. La balance présente donc une latence de mesure de quelques millisecondes.

En réduisant la vitesse de remplissage vers la fin du remplissage, les incertitudes de mesure dues à la latence de la balance peuvent être réduites.

Par ailleurs, l'invention peut être mise en oeuvre pour remplir différents gobelets avec un seul type de médicament. Dans ce mode de réalisation, les patients reçoivent des prescriptions de quantités variables du même médicament.

En variante, plusieurs types de médicaments peuvent être disponibles dans l'automate et ces différents médicaments sont sélectionnés en fonction de la prescription de chaque patient.

Pour ce faire, ledit poste de remplissage comporte préférentiellement plusieurs réservoirs contenant plusieurs médicaments distincts, chaque réservoir étant surmonté d'une pompe et d'une tête de remplissage ;
les réservoirs, les pompes et lesdites têtes de remplissage associés étant montés sur un barillet mobile en rotation ;
l'organe de supervision étant configuré pour commander le déplacement du barillet afin de placer la tête de remplissage du réservoir contenant le médicament prescrit au-dessus du gobelet à remplir.

De préférence, chaque pompe comporte un mécanisme d'accouplement mâle destiné à coopérer avec un mécanisme d'accouplement femelle monté à l'extrémité d'un axe du moteur, l'organe de supervision étant configuré pour commander l'accouplement du moteur avec la pompe du réservoir contenant le médicament prescrit lorsque la tête de remplissage du réservoir contenant le médicament prescrit est placée au-dessus du gobelet à remplir.

Ce mode de réalisation permet d'utiliser un seul moteur pour commander les différentes pompes des différents médicaments et, ainsi, de limiter les pièces d'usures.

Outre le poste de remplissage, l'invention peut également comporter d'autres postes de conditionnement et/ou de contrôle.

De préférence, ledit automate de conditionnement comporte également :
- un poste de dilution ;
- un poste de dépose d'un opercule ;
- un poste de thermoscellage de l'opercule ;
- un poste de marquage de l'opercule ;
- un poste de contrôle et de marquage du gobelet ; et
- un poste de tri des gobelets conformes/non conformes ;
l'organe de supervision étant configuré pour commander le déplacement du convoyeur entre les différents postes.

Le poste de dilution permet d'obtenir une quantité de dilution propre à chaque patient ou de spécifier une quantité minimum de liquide dans chaque gobelet délivré.

Par exemple, une quantité minium de 10 ml peut être programmée dans l'automate de sorte à éviter qu'un gobelet ne contienne que quelques gouttes de médicament. Par ailleurs, le poste de dilution peut éventuellement être associé à un poste de mélangeage.

Les postes de dépose d'un opercule et de de thermoscellage visent à déposer et à fixer un opercule hermétique sur le gobelet pour éviter la contamination ou le renversement du gobelet lors de l'acheminement au patient.

Les postes de marquage de l'opercule et du gobelet visent à indiquer des informations d'identification du patient pour faciliter son acheminement au patient. De préférence, un code 1d ou 2d peut être marqué sur le gobelet, ce code indiquant un numéro d'ordonancier, alors que l'opercule est marqué avec des informations sur la quantité et le médicament remplis dans le gobelet. Ensuite, au niveau du poste de contrôle, un lecteur de code peut lire les codes et comparer la quantité et le médicament remplis dans le gobelet avec la quantité et le médicament prescrit.

Le poste de tri des gobelets conformes/non conformes permet d'évacuer les gobelets suivant leurs états. Pour ce faire, les gobelets conformes sont préférentiellement évacués sur une table rotative et les gobelets non conformes sont déplacés sur une ligne dédiée.

Outre les postes de conditionnement, ledit automate de conditionnement peut également comporter un poste de maintenance de l'au moins un réservoir de médicament, ledit poste de maintenance comportant :
- un lecteur de tag RFID configuré pour lire un tag RFID présent sur l'au moins un réservoir et un tag RFID présent sur un contenant de remplacement du médicament contenu dans le réservoir ;
- une interface homme-machine permettant à un opérateur d'indiquer la quantité de médicament réapprovisionnée dans le réservoir lors d'une phase de remplissage de l'au moins un réservoir de médicament ;
- l'organe de supervision étant configuré pour estimer, lors des phases de remplissage, la quantité restante de médicament dans l'au moins un réservoir de médicament au moyen d'une balance afin d'informer l'opérateur sur l'interface homme-machine lorsque l'au moins un réservoir de médicament présente une quantité de remplissage inférieure à une valeur seuil.

Selon un second aspect, l'invention concerne un procédé de conditionnement de prescriptions mis en oeuvre par un automate de conditionnement selon le premier aspect de l'invention, ledit procédé comportant les étapes suivantes :
- déplacement du convoyeur sous une colonne de stockage de gobelets de sorte qu'un gobelet soit reçu par une ouverture ;
- déplacement du convoyeur entre la colonne de stockage et le poste de remplissage ;
- levée de la balance dans la position de remplissage de sorte que le gobelet soulevé ne soit plus soutenu par le convoyeur ;
- déplacement du barillet pour placer la tête de remplissage du réservoir contenant le médicament prescrit au-dessus du gobelet à remplir ;
- accouplement du moteur avec la pompe du réservoir contenant le médicament prescrit ;
- remplissage du gobelet en fonction du poids mesuré par la balance à force électromagnétique de compensation jusqu'à ce que le poids mesuré corresponde à la quantité prescrite ; et
- désaccouplement du moteur et de la pompe, et descente du plateau dans la position de convoyage.

Selon un mode de réalisation, le procédé comporte également les étapes suivantes :
- déplacement du convoyeur entre le poste de remplissage et un poste de dilution ;
- dilution du médicament prescrit avec une quantité prédéterminée ;
- déplacement du convoyeur entre le poste de dilution et un poste de dépose d'un opercule ;
- dépose de l'opercule ;
- déplacement du convoyeur entre le poste de dépose d'un opercule et un poste de thermoscellage de l'opercule ;
- soudure de l'opercule sur le gobelet ;
- déplacement du convoyeur entre le poste de thermoscellage de l'opercule et un poste de marquage de l'opercule ;
- marquage, sur l'opercule ; d'au moins une information relative au médicament délivré et à la quantité délivré ;
- déplacement du convoyeur entre le poste de marquage de l'opercule et un poste de contrôle et de marquage du gobelet ;
- marquage, sur le gobelet ; d'au moins une information relative au patient ;
- contrôle des informations marquées sur le gobelet et l'opercule avec les informations contenues dans la base de données ; et
- déplacement du convoyeur entre le poste de contrôle et de marquage du gobelet et un poste de tri des gobelets conformes/non conformes.

### DESCRIPTION DES FIGURES

La manière de réaliser l'invention, ainsi que les avantages qui en découlent, ressortiront bien de la description des modes de réalisation qui suivent, à l'appui des figures annexées dans lesquelles :
La figure 1 est une représentation schématique d'un automate de conditionnement selon un premier mode de réalisation de l'invention ;
La figure 2 est une vue en perspective d'un automate de conditionnement selon un second mode de réalisation de l'invention ;
La figure 3 est une vue en coupe de dessus de l'automate de conditionnement de la figure 2 ;
La figure 4 est une vue de dessus du convoyeur et du barillet de l'automate de conditionnement de la figure 2 ;
La figure 5 est une vue en perspective du barillet de l'automate de conditionnement de la figure 2 ;
La figure 6 est une vue de côté du poste de remplissage de l'automate de conditionnement de la figure 2 ; et
La figure 7 est un organigramme des étapes de conditionnement mises en oeuvre par un organe de supervision de l'automate de conditionnement de la figure 2.

### DESCRIPTION DETAILLEE DE L'INVENTION

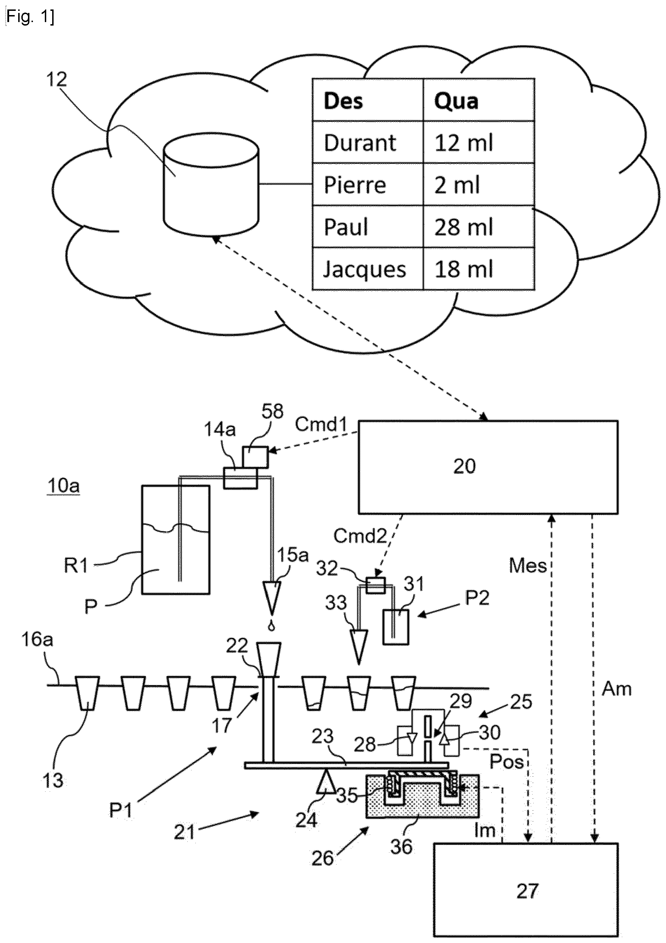

La figure 1 illustre un premier mode de réalisation d'un automate de conditionnement **10a** dans lequel un seul médicament **P** est distribué à différents patients **Des** en fonction de quantités **Qua** prescrites à ces patients **Des.** Pour ce faire, une base de données **12** contient la liste des différents patients **Des** et des quantités Qua de médicaments **P** à conditionner, c'est-à-dire à préparer dans des gobelets **13** individualisés.

Cette base de données **12** peut être stockée dans l'automate **10a** ou accessible à distance par l'automate, par exemple sur un serveur disposé sur Internet et connecté à l'automate **10a**, tel qu'illustré sur la figure 1.

Le médicament **P** est stocké, sous forme liquide, au niveau d'un poste de remplissage **P1** comportant un seul réservoir **R1**. Une pompe **14a** est commandée par un moteur **58** pour pomper une partie du médicament **P** contenue dans le réservoir **R1** et l'acheminer jusqu'à une tête de délivrance **15a**.

Les gobelets **13** sont placés sous la tête de délivrance **15a** au moyen d'un convoyeur **16a**. Ce convoyeur **16a** peut prendre différentes formes telles qu'un barillet, un plateau ou encore un tapis roulant. De préférence, les gobelets **13** présentent une forme tronconique coopérant avec des ouvertures **17** du convoyeur **16a** pour assurer le déplacement des gobelets **13**. Pour ce faire, les ouvertures **17** présentent une forme circulaire avec un diamètre compris entre le diamètre des gobelets **13** au niveau de l'extrémité inférieure et le diamètre des gobelets **13** au niveau de l'extrémité supérieure. En variante, l'extrémité des gobelets **13** peut présenter un rebord avec un diamètre supérieur au diamètre des ouvertures **17**.

L'invention vise à conditionner les quantités **Qua** de médicaments **P** prescrites à chaque patient **Des**. Pour ce faire, un organe de supervision **20** commande le moteur **58** jusqu'à ce que la quantité délivrée dans le gobelet **13** disposé sous la tête de distribution **15a** corresponde à la quantité prescrite **Qua**. L'organe de supervision **20** peut correspondre à un ordinateur, un processeur ou un microcontrôleur ou un automate.

Pour s'assurer de la quantité **Qua** de médicament **P** délivré, l'invention propose d'utiliser une balance à force électromagnétique de compensation **21** disposée sous la tête de délivrance **15a** mobile entre deux positions grâce à un actionneur, non représenté, par exemple un vérin.

Dans une première position, dite position de convoyage, la balance **21** n'interfère pas le déplacement du convoyeur **16a**, c'est-à-dire que le plateau **22** de la balance **21** est disposé sous les gobelets **13** portés par le convoyeur **16** sans entrer en contact avec les gobelets **13**.

Dans une seconde position, dite position de remplissage, la balance **21** soulève un gobelet **13** en direction de la tête de délivrance **15a** de sorte que la balance à force électromagnétique de compensation **21** puisse mesurer l'évolution du poids du gobelet **13** au cours du remplissage de celui-ci. Dans cette position de remplissage, illustrée sur la figure 1, le gobelet **13** est uniquement soutenu par le plateau **22** de la balance **21**.

L'organe de supervision **20** peut donc analyser, au cours de la phase de remplissage, l'évolution du poids du gobelet **13** pour commander le moteur **58** jusqu'à ce que le poids mesuré **Mes** corresponde à la quantité prescrite.

La balance à force électromagnétique de compensation **21** comporte préférentiellement un levier **23** montée sur un pivot **24**. Une première extrémité du levier **23**, disposé à gauche sur la figure 1, reçoit le plateau **22** alors qu'une seconde extrémité, disposé à droite sur la figure 1, coopère avec une cellule de détection de la déviation **25** est un contrepoids électromagnétique **26**.

Dans la phase de détection, c'est-à-dire lorsque le plateau **22** est disposé dans la position de remplissage et soulève le gobelet **13**, l'organe de supervision **20** commande, par le signal **Am**, l'activation de la compensation électromagnétique. Cette compensation électromagnétique est commandée par un régulateur **27** connecté à la cellule de détection de la déviation **25** et au contrepoids électromagnétique **26.** Lorsque la cellule **25** détecte une variation de la position **Pos** du levier **23**, le régulateur **27** commande l'énergie électrique du contrepoids électromagnétique **26** pour s'opposer à la déviation détectée par la cellule **25**.

Par exemple, la cellule **25** peut comporter une source lumineuse **28** et une photodiode **30** disposées en regard l'une de l'autre et fixes par rapport à l'automate **10a**. Entre ces deux composants optoélectroniques, un obturateur est monté sur la seconde extrémité du levier **23** de sorte à être immobile par rapport aux composants optoélectroniques. Cet obturateur comporte une ouverture **29** disposée entre la source lumineuse **28** et la photodiode **30** lorsque le levier **23** est dans une position d'équilibre. Ainsi, l'intensité lumineuse captée par la photodiode **30** est maximum lorsque le levier **23** est dans une position d'équilibre et décroît fortement lorsque qu'une déviation du levier **23** entraîne un décalage de l'ouverture **29** par rapport à l'axe optique entre la source lumineuse **28** et la photodiode **30**. La cellule **25** permet donc de mesurer la position **Pos** du levier **23** en fonction de l'intensité lumineuse captée par la photodiode **30**.

Ainsi, au cours du remplissage du gobelet **13**, la cellule **25** détecte les déplacements très faibles de l'ouverture **29** et le régulateur **27** compense ces déplacements en augmentant intensité **Im** de l'énergie électrique appliquée sur le contrepoids électromagnétique **26**.

Pour ce faire, le contrepoids électromagnétique **26** peut comporter un aimant permanent **36** et une bobine **35** dont le courant est commandé par le régulateur **27**. L'intensité **Im** du courant nécessaire pour obtenir la stabilité du levier **23** est également analysée par le régulateur **27** pour transmettre un signal de mesure **Mes** du poids des éléments disposés sur le plateau **22**.

L'organe de supervision **20** peut donc commander le régulateur **27**, au moyen d'un signal d'activation **Am**, pour obtenir une régulation de la position du levier **23** par rapport à une position d'équilibre dans la position de remplissage. De préférence, la phase de remplissage débute par une première phase de mesure du poids à vide du gobelet par la balance **21** afin de réaliser une tare du système de pesée. En variante, le poids du gobelet **13** peut-être préenregistrés dans une mémoire de l'organe de supervision **20**.

Avec cette mesure **Mes** issue de la balance **21**, l'organe de supervision **20** peut déduire le poids de médicament **P** distribué dans le gobelet **13** au cours de la phase de remplissage à partir de la mesure du poids sur le plateau **22**. En connaissant la masse volumique du médicament **P**, l'organe de supervision **20** peut convertir le poids estimé de médicament **P** délivré dans le gobelet **13** en une information de quantité de médicament **P** délivrée. En comparant la quantité de médicament **P** délivrée avec la quantité prescrite **Qua**, l'organe de supervision **20** peut asservir le fonctionnement de la pompe **14a** actionnée par un moteur **58**, au moyen d'un signal **Cmd1,** pour atteindre la quantité prescrite **Qua**. Compte tenu de la latence de détection de la balance **21**, c'est-à-dire du temps nécessaire pour que la balance **21** effectue la régulation de l'intensité électrique **Im** nécessaire pour atteindre l'équilibre du levier **23**, l'organe de supervision **20** peut commander la pompe **14a** pour délivrer le médicament **P** selon plusieurs vitesses : une première vitesse rapide pour que la quantité de médicaments délivrée atteigne 80 % de la quantité prescrite **Qua** ; et une seconde vitesse plus lente pour les dernières gouttes de médicament. Il est ainsi possible d'obtenir la quantité prescrite **Qua** à la goutte près.

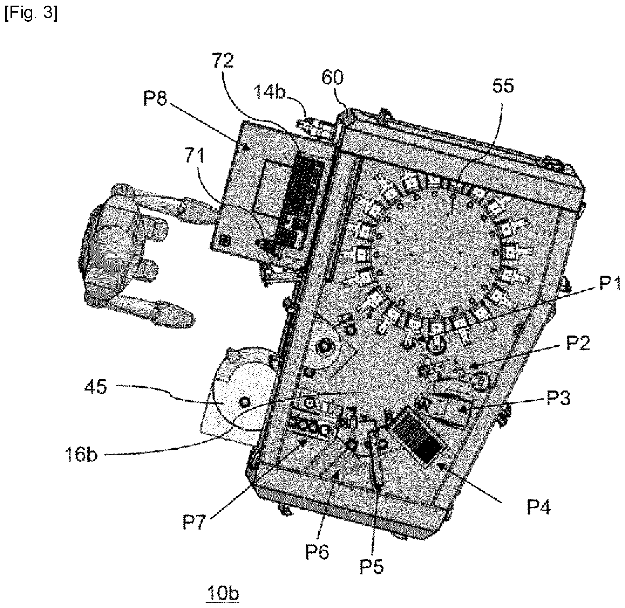

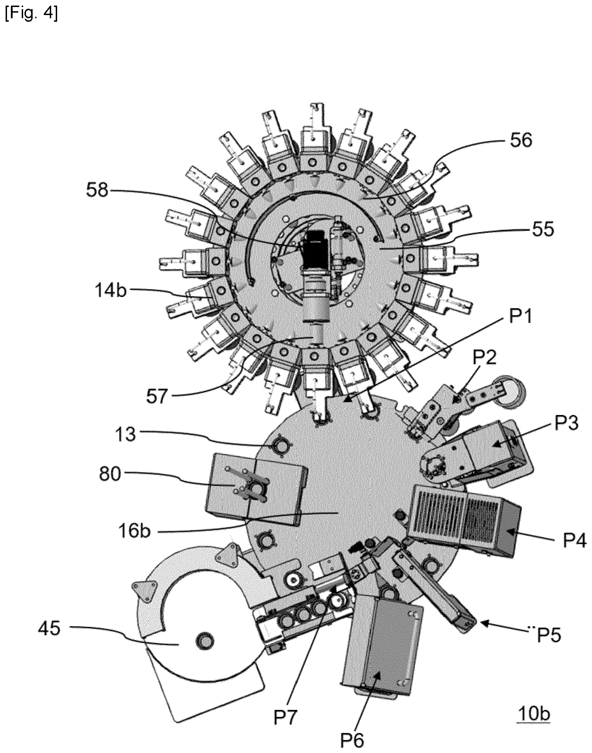

Lorsque le remplissage de la quantité prescrite **Qua** est terminé, le plateau **22** est redescendu en déplaçant le plateau **22** dans la position de convoyage. Le gobelet rempli **13** se retrouve donc supporté par le convoyeur **16a** et peut être déplacé dans différents postes **P2-P7** de préparation du gobelet **13** en vue de sa délivrance au patient **Des**, tel qu'illustré sur les figures 3 et 4. Par exemple, tel qu'illustré sur la figure 1, un poste **P2** peut permettre la dilution du médicament **P**, notamment lorsque la quantité de médicaments prescrites **Qua** est très faible. En effet, s'il est prescrit trois gouttes de médicaments **P**, il peut être compliqué d'administrer ces trois gouttes de médicaments **P** et la dilution de ce médicament **P** peut permettre son administration complète.

Pour effectuer la dilution, la figure 1 illustre un réservoir **31** comportant un produit de dilution, par exemple de l'eau, un sérum physiologique ou une eau glucosée, relié à une tête de dilution **33** par une pompe **32**.

La pompe **32** est commandée par l'organe de supervision **20**, au moyen d'un signal de commande **Cmd2**, pour obtenir la dilution recherchée. Ainsi, la base de données **12** peut intégrer également une information sur une quantité de dilution recherchée pour chaque médicament **P**. En variante, l'organe de supervision **20** peut être programmé pour que tous les gobelets soient délivrés avec une quantité équivalents, par exemple 30 ml. La quantité de dilution peut donc être déterminée en calculant la différence entre 30 ml et la quantité prescrite **Qua**.

La quantité de produits de dilution peut simplement être asservie par une pompe classique lorsque la quantité de dilution n'est pas sensible, tel qu'illustré sur la figure 1. Dans le cas contraire, il est possible d'utiliser également une balance de précision pour mesurer la quantité de délivrance du produit de dilution.

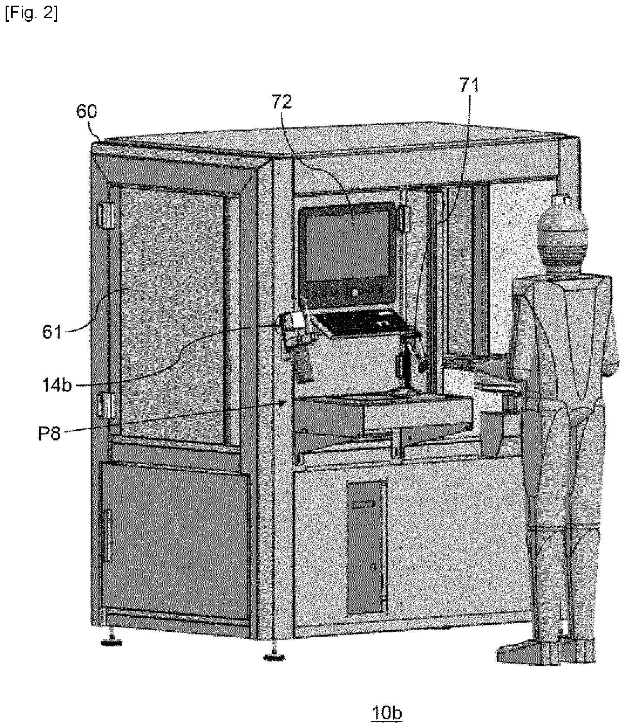

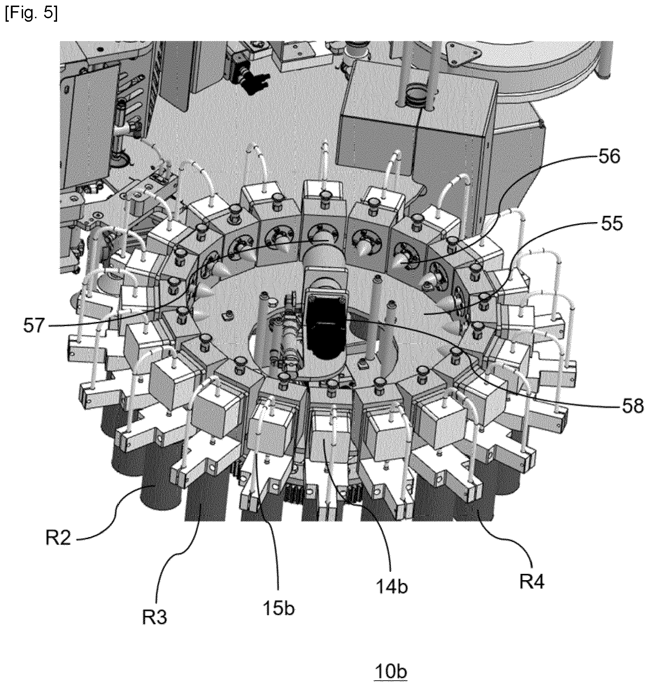

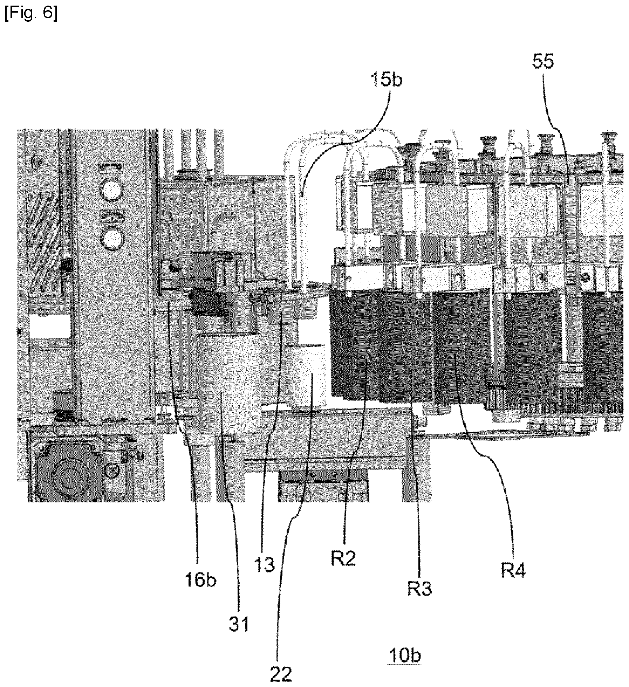

D'autres postes de traitement des gobelets **13** peuvent également être mis en place après avoir procédé au remplissage de la quantité prescrite **Qua.** Par exemple, les figures 2 à 6 illustrent un second mode de réalisation dans lequel un automate de conditionnement **12b** comporte un poste de dilution **P2**, un poste de dépose d'un opercule **P3**, un poste de thermoscellage de l'opercule **P4**, un poste de marquage de l'opercule **P5**, un poste de contrôle et de marquage du gobelet **P6** et un poste de tri des gobelets conformes/non conformes **P7**.

L'automate **10b** comporte un châssis **60** protégeant les opérations de remplissage des contaminations extérieures. Pour pouvoir intervenir à l'intérieur du châssis, des ouvertures **61** sont prévues dans le châssis **60.**

À l'extérieur du châssis **60,** l'automate **10b** présente un poste de maintenance **P8** intégrant un lecteur de tag RFID **71** et une interface homme-machine **72**. Le poste de maintenance **P8** est destiné au contrôle des opérations de remplissage et au réapprovisionnement des réservoirs **R2-R4** de médicaments **P** contenues dans l'automate **10b**.

Pour lancer des opérations de conditionnement, l'opérateur peut sélectionner sur l'interface homme-machine **72** la liste des patients et les prescriptions associées qu'il souhaite conditionner. Par exemple, l'opérateur peut sélectionner l'ensemble des résidents d'une maison de retraite où l'ensemble des patients d'une unité de soins particulières d'un hôpital.

En outre, sur l'interface homme-machine **72**, l'opérateur peut contrôler l'état de remplissage des réservoirs **R2-R4** de médicaments **P**. Par exemple, le niveau de remplissage des réservoirs **R2-R4** peut être contrôlé par une balance associée à chaque réservoir **R2-R4** ou une balance unique configurée pour mesurer le poids du réservoir **R2-R4** sélectionné au niveau du poste de remplissage **R1**.

Lorsque l'opérateur détecte qu'un niveau de remplissage est trop bas pour effectuer les conditionnements prescrits, il peut ouvrir l'automate **10b** pour extraire les réservoirs **R2-R4** à réapprovisionner. Le réservoir est alors posé à gauche du poste de remplissage **P8** après avoir été scanné par le lecteur de tag RFID **71.** Dès qu'un réservoir **R2-R4** est scanné par le lecteur de tag RFID **71**, l'organe de supervision **20** identifie que l'opérateur veut procéder à un réapprovisionnement de ce réservoir **R2-R4.**

L'opérateur peut ensuite scanner un tag RFID d'un contenant de remplacement de médicament au moyen du lecteur de tag RFID 71. L'organe de supervision peut alors contrôler que le médicament réapprovisionné correspond bien au médicament **P** préalablement stocké dans le réservoir **R2-R4**.

En variante, l'opérateur a également la possibilité de vider et de nettoyer le réservoir **R2-R4** pour réapprovisionner un autre type de médicament **P** afin de modifier les possibilités de délivrance de l'automate **10b**. De même, l'opérateur peut avoir à sa disposition une armoire avec un grand nombre de réservoirs **R2-R4** contenant des médicaments **P** distincts qu'il peut agencer dans l'automate **10b** en fonction des prescriptions qu'il souhaite conditionner.

Lorsque le réservoir **R2-R4** placé au niveau du poste de maintenance **P8** est rempli, une balance peut mesurer la quantité de médicament **P** réapprovisionnée dans le réservoir **R2-R4** afin que l'organe de supervision **20** puisse estimer, au cours des phases de remplissage utilisant ce médicament **P,** la quantité restante de produits **P** dans le réservoir **R2-R4**.

En outre, même si un réservoir **R2-R4** venait à se vider plus vite que la quantité restante estimée, l'organe de supervision **20** est configuré pour détecter, lors d'une phase de remplissage, qu'il n'est pas possible d'atteindre la quantité prescrite. Par exemple, si la commande de remplissage de la pompe **14b** ne permet pas d'augmenter le poids mesuré par la balance **21**, l'organe de supervision peut demander à l'opérateur de remplacer le réservoir **R2-R4** utilisé au cours de la phase de remplissage.

Contrairement à l'automate **10a** de la figure 1, l'automate 10b illustré sur les figures 2 à 6 propose d'intégrer plusieurs réservoirs **R2-R4** distincts à l'intérieur de l'automate **10b**. Le poste de remplissage **P1** permet donc également à l'organe de supervision **20** de sélectionner le médicament **P** à conditionner. Ainsi, la base de données **12** contient également une information sur le type de médicament **P** à conditionner pour chaque patient **Des**.

Tel qu'illustré sur les figures 3 à 5, les différents réservoirs **R2 R4** sont montés sur un barillet **55** commandé en rotation de sorte à placer la tête de remplissage **15b** du réservoir **R2-R4** contenant le médicament **P** prescrit au-dessus du gobelet **13** à remplir. Chaque contenant **R2-R4** est associé à une pompe **14b** et une tête de délivrance **15b**. L'activation de la pompe **14b** du médicament **P** sélectionné est préférentiellement réalisée au moyen d'un seul moteur **58** commandé par l'organe de supervision **20**. Ainsi, un système d'accouplement mâle **56** est disposé sur l'axe de chaque pompe **14b** et ce système d'accouplement mâle **56** est destiné à coopérer avec un système d'accouplement femelle **57** solidaire de l'axe du moteur **58**.

Lorsque le médicament **P** a été sélectionné, le réservoir contenant ce médicament **P est** déplacé en regard du gobelet **13** à remplir et l'axe du moteur **58** est déplacé pour réaliser un accouplement entre le moteur **58** et la pompe 10b du réservoir **R2-R4** sélectionné.

Le convoyeur **16b** illustré sur les figures 3 à 5 se présente également sous la forme d'un barillet. Ce convoyeur **16b** permet de conditionner plusieurs gobelets **13** simultanément en déplaçant ces gobelets **13** entre les différents postes **P1-P7** de conditionnement.

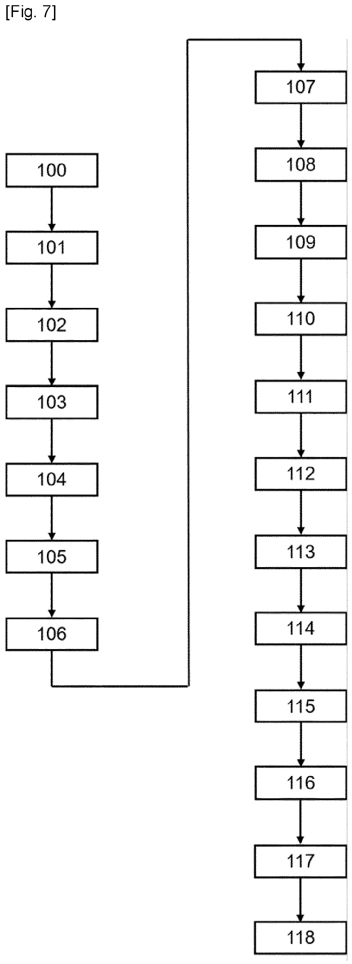

Les mouvements du convoyeur **16b** et du barillet **55** portant les réservoirs **R2-R4** sont décrits en référence à la figure 7 illustrant le procédé de conditionnement mis en oeuvre par l'organe de supervision **20** de l'automate **10b**.

Le programme intégré dans l'organe de supervision **20** comporte une première étape **100** de positionnement d'une ouverture **17** du convoyeur **16b** en regard d'une colonne de stockage **80** de gobelets **13** vides. Une seconde étape **101** consiste à déplacer le convoyeur **16b** de sorte qu'un gobelet **13** soit positionné sous la tête de délivrance **15b** du réservoir **R2-R4** associé au médicament **P** à conditionner.

En parallèle de ces deux premières étapes **100-101,** l'organe de supervision **20** récupère la prescription dans la base de données **12**, c'est-à-dire les informations sur le patient **Des** et sur la nature et la quantité de médicament **P** à conditionner, de sorte à associer chaque gobelet **13** déplacé par le convoyeur **16b** à un patient **Des** particulier.

Ainsi, à l'issue de l'étape **101**, le gobelet **13** d'un patient particulier est placé au niveau du poste de remplissage **P1**. Dans l'étape **102**, l'organe de supervision **20** commande la levée de la balance **21**.

À partir de la prescription destinée au patient **Des** pour lequel le gobelet **13** doit être acheminé, l'organe de supervision **20** peut déplacer le barillet **55** pour sélectionner le réservoir **R2-R4** à utiliser, dans l'étape **103**. Lorsque la tête de délivrance **15b** du réservoir **R2-R4** contenant le médicament **P** prescrit est placée au-dessus du gobelet **13**, l'organe de supervision **20** commande l'accouplement du moteur **58** avec la pompe **14b** du réservoir **R2-R4** sélectionné, dans l'étape **104**.

Dans l'étape **105**, l'organe de supervision **20** commande le remplissage du gobelet **13** au moyen d'un contrôle de l'évolution du poids mesuré **Mes** par la balance **21**.

En effet, au cours de la phase de remplissage, le poids mesuré **Mes** par la balance **21** est transmis à l'organe de supervision **20** qui convertit ce poids **Mes** en une quantité de médicament **P** délivrée, connaissant le poids à vide du gobelet **13** et la masse volumique du médicament **P**.

Lorsque la quantité de médicament **P** prescrite a été remplie dans le gobelet **13**, l'organe de supervision **20** stoppe le remplissage du gobelet 13 et, dans l'étape **106**, le moteur **58** est désaccouplé de la pompe **14b** pendant que le plateau **22** est déplacé dans la position de convoyage. À l'issue de cette étape **106**, le gobelet **13** est rempli et celui-ci est à nouveau porté par le convoyeur 16b.

Le convoyeur **16b** peut ensuite entraîner le gobelet **13** dans les autres postes de conditionnement **P2-P7**. Tel qu'illustré sur les figures 1 à 6, un second poste de conditionnement **P2** permet de réaliser la dilution du médicament **P**. Pour ce faire, l'organe de supervision **20** déplace le gobelet **13** entre le poste remplissage **P1** et le poste de dilution **P2**, dans une étape **107**, et il commande la pompe de dilution **32** pour délivrer une quantité prédéterminée d'un produit de dilution, dans une étape **108**.

De même, pour procéder à la fermeture du gobelet **13**, l'organe de supervision **20** déplace le convoyeur **16b** entre le poste de dilution **P2** et le poste de dépose d'un opercule **P3**, dans une étape **109**, et il commande la pose de l'opercule, dans une étape **110**.

Après le poste de dépose d'un opercule **P3**, l'organe de supervision **20** déplace le convoyeur **16b** pour atteindre un poste de thermoscellage de l'opercule **P4**, dans une étape **111**. L'étape **112** consiste à réaliser la fixation de l'opercule sur le gobelet **13** alors que les étapes **113** et **114** correspondent au déplacement et au marquage l'opercule avec des informations relatives au médicament **P** délivré et à la quantité délivré **Qua**, dans un poste de marquage de l'opercule **P5**. Cette étape de marquage est préférentiellement réalisée avec un code d'identification, par exemple numéro d'ordonnancier.

Ensuite, l'organe de supervision **20** procède au déplacement **115** et au marquage **116** du gobelet **13** avec une information relative au patient **Des**, au niveau du poste de marquage du gobelet **P6**.

Au niveau du poste de marquage du gobelet **P6**, l'organe de supervision **20** procède également au contrôle des informations présentes sur l'opercule et le gobelet **13**. Ainsi, ces informations peuvent être comparées aux informations attendues, c'est-à-dire à la prescription stockée dans la base de données **12**. Si une erreur a été commise au niveau du poste de remplissage **P1**, par exemple parce que, lors du remplissage, l'opérateur a été contraint de réapprovisionner le réservoir **R2-R4** utilisé pour le remplissage et il a commis une erreur, cette erreur devrait être détectée au niveau du poste de contrôle **P6**, dans l'étape **117**.

Après ce poste de contrôle **P6**, si une erreur est détectée entre la prescription et les éléments effectivement marqués sur le gobelet **13**, ce gobelet **13** est mis au rebut par un poste de tri des gobelets conformes/non conformes **P7** et un nouveau cycle de conditionnement pourra être réalisé pour le patient **Des** associé à ce gobelet **13**. En pratique, le gobelet **13** est pratiquement toujours conforme à la prescription si bien que, dans l'étape **118**, le gobelet **13** est très souvent acheminé vers un plateau de sortie **45** de l'automate **10b**. L'opérateur peut ensuite acheminer aux patients **Des** l'ensemble des gobelets **13** préparés et délivrés sur le plateau de sortie **45**.

L'invention permet, ainsi, de conditionner des médicaments **P** sous forme liquide avec un contrôle très précis de la quantité de médicaments **P** contenus dans les gobelets **13**. En outre, une traçabilité très stricte est appliquée sur les gobelets **13** permettant de limiter au maximum les risques de fausses médications.

Par ailleurs, avec le conditionnement par postes **P1-P7** des gobelets **13**, il est possible de conditionner simultanément des gobelets **13** sur les différents postes **P1-P7** afin d'atteindre une cadence de conditionnement importante, par exemple entre 300 et 400 gobelets **13** par heure.

## Revendications

1. Automate de conditionnement (10a-10b) de prescriptions pour un ensemble de patients (Des), au moyen d'une base de données (12) comportant une quantité prescrite (Qua) d'un médicament (P) associé à chaque patient (Des), ledit automate de conditionnement (10a-10b) comportant :
- des gobelets (13) destinés à être délivrés aux différents patients (Des) avec la quantité prescrite (Qua) de médicament (P) ;
- un convoyeur (16a-16b) de déplacement des gobelets (13) comportant des ouvertures traversantes (17) permettant de recevoir les gobelets (13) afin de les déplacer jusqu'à un poste de remplissage (P1) comportant :
▪ au moins un réservoir (R1-R4) de médicament (P) ;
▪ une tête de remplissage (15a-15b) destinée à délivrer le médicament (P) ;
▪ un pompe (14a-14b) connectée entre le réservoir (R1-R4) et la tête de remplissage (15a-15b) ; et
▪ un moteur (58) actionnant la pompe de sorte à délivrer le médicament (P) contenu dans le réservoir (R1-R4) au niveau de la tête de remplissage (15a-15b) ; et
- un organe de supervision (20), connecté à la base de données (12), configuré pour commander la pompe (14a-14b) de sorte à délivrer la quantité prescrite (Qua) ; ***caractérisé en ce que*** ledit poste de remplissage (P1) comporte également :
- une balance à force électromagnétique de compensation (21) comportant un plateau (22), et un actionneur de déplacement de ladite balance (21) configuré pour déplacer ladite balance (21) entre deux positions :
▪ une position de remplissage dans laquelle le plateau (22) soulève un gobelet (13) vers ladite tête de délivrance (15a-15b) de sorte que le gobelet (13) soulevé ne soit plus soutenu par le convoyeur (16a-16b) et que la balance (21) puisse mesurer l'évolution du poids (Mes) du gobelet (13) soulevé au cours du remplissage de celui-ci ; et
▪ une position de convoyage dans laquelle ledit plateau (22) n'est pas au contact d'un gobelet (13) de sorte que le convoyeur (16a-16b) puisse déplacer les gobelets (13) ;
- ledit organe de supervision (20) étant configuré pour commander le moteur (58) en fonction du poids mesuré (Mes) par ladite balance à force électromagnétique de compensation (21) jusqu'à ce que le poids mesuré (Mes) corresponde à la quantité prescrite (Qua).

2. Automate de conditionnement selon la revendication 1, ***dans lequel*** ladite balance à force électromagnétique de compensation (21) comporte :
- un levier (23) à une première extrémité duquel le plateau (22) est monté ;
- une cellule de détection de la déviation (25) montée sur une seconde extrémité du levier (23) et configurée pour détecter une déviation (Pos) du levier (23) d'une position d'équilibre ;
- un pivot (24) placé sous le levier (23) entre la première et la seconde extrémité de sorte que les déplacements du plateau (22) dus à la force de gravité entraînent un déplacement d'au moins une partie de la cellule de détection de la déviation (25) ;
- un contrepoids électromagnétique (26) disposé sous le levier (23) au niveau de la seconde extrémité ; et
- un régulateur (27), connecté à la cellule de détection de la déviation (25) et au contrepoids électromagnétique (26) ;
- le régulateur (27) étant configuré pour commander, dans la position de remplissage, l'énergie électrique (Im) du contrepoids électromagnétique (26) de sorte à s'opposer à une déviation (Pos) détectée par la cellule de détection de la déviation (25), le poids (Mes) du gobelet (13) étant calculé en fonction de l'énergie électrique (Im) imposée au contrepoids électromagnétique (26) pour s'opposer à une déviation (Pos) dudit levier (23).

3. Automate de conditionnement selon la revendication 2, ***dans lequel*** ladite cellule de détection de la déviation (25) comporte :
- une source lumineuse (28) et une photodiode (30), montées en regard l'une de l'autre et fixes par rapport à un carter (60) de l'automate de conditionnement (10a-10b) ; et
- un obturateur monté sur la seconde extrémité du levier (23), ledit obturateur étant muni d'une ouverture (29) disposée entre la source lumineuse (28) et la photodiode (30) lorsque le levier (23) est dans la position d'équilibre.

4. Automate de conditionnement selon l'une des revendications 1 à 3, ***dans lequel*** ledit organe de supervision (20) est configuré pour commander la pompe (14a-14b) avec une course non linéaire comportant au moins deux vitesses de remplissage, une première vitesse jusqu'à atteindre au moins 80% de la quantité prescrite (Qua) et une seconde vitesse, inférieure à la première vitesse, jusqu'à atteindre 100% de la quantité prescrite (Qua).

5. Automate de conditionnement selon l'une des revendications 1 à 4, ***dans lequel*** ledit poste de remplissage (P1) comporte plusieurs réservoirs (R2-R4) contenant plusieurs médicaments (P) distincts, chaque réservoir (R2-R4) étant surmonté d'une pompe (14b) et d'une tête de remplissage (15b) ;
les réservoirs (R2-R4), les pompes (14b) et lesdites têtes de remplissage (15a-15b) associés étant montés sur un barillet (55) mobile en rotation ;
l'organe de supervision (20) étant configuré pour commander le déplacement du barillet (55) afin de placer la tête de remplissage (15b) du réservoir (R2-R4) contenant le médicament (P) prescrit au-dessus du gobelet (13) à remplir.

6. Automate de conditionnement selon la revendication 5, ***dans lequel*** chaque pompe (14b) comporte un mécanisme d'accouplement mâle (56) destiné à coopérer avec un mécanisme d'accouplement femelle (57) monté à l'extrémité d'un axe du moteur (58), l'organe de supervision (20) étant configuré pour commander l'accouplement du moteur (58) avec la pompe (14b) du réservoir (R2-R4) contenant le médicament (P) prescrit lorsque la tête de remplissage (15b) du réservoir (R2-R4) contenant le médicament (P) prescrit est placée au-dessus du gobelet (13) à remplir.

7. Automate de conditionnement selon l'une des revendications 1 à 6, ***dans lequel*** ledit automate de conditionnement (10a-10b) comporte également :
- un poste de dilution (P2) ;
- un poste de dépose d'un opercule (P3) ;
- un poste de thermoscellage de l'opercule (P4) ;
- un poste de marquage de l'opercule (P5) ;
- un poste de contrôle et de marquage du gobelet (P6) ; et
- un poste de tri des gobelets conformes/non conformes (P7) ;
l'organe de supervision (20) étant configuré pour commander le déplacement du convoyeur (16a-16b) entre les différents postes (P1-P7).

8. Automate de conditionnement selon l'une des revendications 1 à 7, ***dans lequel*** ledit automate de conditionnement (10a-10b) comporte également un poste de maintenance (P8) de l'au moins un réservoir (R1-R4) de médicament (P), ledit poste de maintenance (P8) comportant :
- un lecteur de tag RFID (71) configuré pour lire un tag RFID présent sur l'au moins un réservoir (R1-R4) et un tag RFID présent sur un contenant de remplacement du médicament (P) contenu dans le réservoir (R1-R4) ; et
- une interface homme-machine (72) permettant à un opérateur d'indiquer la quantité de médicament (P) réapprovisionnée dans le réservoir (R1-R4) lors d'une phase de remplissage de l'au moins un réservoir (R1-R4) de médicament (P) ;
- l'organe de supervision (20) étant configuré pour estimer, lors des phases de remplissage, la quantité restante de médicament (P) dans l'au moins un réservoir (R1-R4) de médicament (P) au moyen d'une balance afin d'informer l'opérateur sur l'interface homme-machine (72) lorsque l'au moins un réservoir (R1-R4) de médicament (P) présente une quantité de remplissage inférieure à une valeur seuil.

9. Procédé de conditionnement de prescriptions mis en oeuvre par un automate de conditionnement (10a-10b) selon la revendication 7, ledit procédé comportant les étapes suivantes :
- déplacement (100) du convoyeur (16a-16b) sous une colonne (80) de stockage de gobelets (13) de sorte qu'un gobelet (13) soit reçu par une ouverture (17) ;
- déplacement (101) du convoyeur (16a-16b) entre la colonne (80) de stockage et le poste de remplissage (P1) ;
- levée (102) de la balance (21) dans la position de remplissage de sorte que le gobelet (13) soulevé ne soit plus soutenu par le convoyeur (16a-16b) ;
- déplacement (103) du barillet (55) pour placer la tête de remplissage (15b) du réservoir (R1-R4) contenant le médicament (P) prescrit au-dessus du gobelet (13) à remplir ;
- accouplement (104) du moteur (58) avec la pompe (14b) du réservoir (R1-R4) contenant le médicament (P) prescrit ;
- remplissage (105) du gobelet (13) en fonction du poids mesuré (Mes) par la balance à force électromagnétique de compensation (21) jusqu'à ce que le poids mesuré (Mes) corresponde à la quantité prescrite (Qua) ; et
- désaccouplement (106) du moteur (58) et de la pompe (14b) et descente du plateau (22) dans la position de convoyage.

10. Procédé de conditionnement selon la revendication 9, *dans lequel* le procédé comporte également les étapes suivantes :
- déplacement (107) du convoyeur (16a-16b) entre le poste de remplissage (P1) et un poste de dilution (P2) ;
- dilution (108) du médicament (P) prescrit avec une quantité prédéterminée ;
- déplacement (109) du convoyeur (16a-16b) entre le poste de dilution (P2) et un poste de dépose d'un opercule (P3) ;
- dépose (110) de l'opercule ;
- déplacement (111) du convoyeur (16a-16b) entre le poste de dépose d'un opercule (P3) et un poste de thermoscellage de l'opercule (P4) ;
- soudure (112) de l'opercule sur le gobelet (13) ;
- déplacement (113) du convoyeur (16a-16b) entre le poste de thermoscellage de l'opercule (P4) et un poste de marquage de l'opercule (P5) ;
- marquage (114), sur l'opercule; d'au moins une information relative au médicament (P) délivré et à la quantité délivré (Qua) ;
- déplacement (115) du convoyeur (16a-16b) entre le poste de marquage de l'opercule (P5) et un poste de contrôle et de marquage du gobelet (P6) ;
- marquage (116), sur le gobelet (13); d'au moins une information relative au patient (Des) ;
- contrôle (117) des informations marquées sur le gobelet (13) et l'opercule avec les informations contenues dans la base de données (12) ; et
- déplacement (118) du convoyeur (16a-16b) entre le poste de contrôle et de marquage du gobelet (P6) et un poste de tri des gobelets conformes/non conformes (P7).

## Patentansprüche

1. Verpackungsautomat (10a-10b) für verschriebene Medikamente für ein Gruppe von Patienten (Des), mit Hilfe einer Datenbank (12), der eine verschriebene Menge (Qua) eines Medikamentes (P) für den einzelnen Patienten (Des) enthält, dieser Verpackungsautomat (10a-10b) umfasst:
- Becher (13), vorgesehen zur Ausgabe an die verschiedenen Patienten (Des) mit der verschriebenen Menge (Qua) des Medikamentes (P);
- eine Beförderungsvorrichtung (16a-16b) zum Verschieben der Becher (13), die durchführende Öffnungen (17) enthält, um die Becher (13) aufnehmen zu können, damit sie zu einer Befüllstation (PI) geschoben werden können, die umfasst
▪ mindestens einen Vorratsbehälter (R1-R4) des Medikamentes (P);
▪ einen Füllkopf (15a-15b), vorgesehen zur Abgabe des Medikamentes (P);
▪ eine Pumpe (14a-14b), verbunden zwischen dem Vorratsbehälter (R1-R4) und dem Füllkopf (15a-15b); und
▪ einen Motor (58) zur Betätigung der Pumpe, um so das Medikament (P) abzugeben, das im Vorratsbehälter (R1-R4) enthalten ist, in Höhe des Füllkopfes (15a-15b); und
- eine Überwachungsvorrichtung (20), verbunden mit der Datenbank (12), konfiguriert zur Steuerung der Pumpe (14a-14b) um die verschriebene Menge (Qua) abzugeben; ***dadurch gekennzeichnet, dass*** diese Befüllstation (PI) außerdem enthält:
- eine elektromagnetische Kraftkompensationswaage (21) mit einer Platte (22), und einem Stellglied zum Verschieben dieser Waage (21) konfiguriert zum Verschieben dieser Waage (21) zwischen zwei Positionen:
▪ einer Füllposition, in der die Platte (22) einen Becher (13) zu diesem Füllkopf (15a-15b) hebt, so dass dieser angehobene Becher (13) nicht länger von der Beförderungsvorrichtung (16a-16b) gestützt wird und die Waage (21) die Gewichtsveränderung (Mes) des angehobenen Bechers (13) während des Füllens messen kann; und
▪ einer Transportposition, bei der diese Platte (22) keinen Kontakt mit dem Becher (13) hat, so dass die Beförderungsvorrichtung (16a-16b) die Becher (13) verschieben kann;
- diese Überwachungsvorrichtung (20) ist dazu konfiguriert, den Motor (58) entsprechend dem von dieser elektromagnetischen Kraftkompensationswaage (21) gemessenen Gewicht (Mes) zu steuern, bis das gemessene Gewicht (Mes) der verschriebenen Menge (Qua) entspricht.

2. Verpackungsautomat nach Anspruch 1, *bei dem* diese elektromagnetische Kraftkompensationswaage (21) umfasst:
- einen Hebel (23), an dessen erstem Ende die Platte (22) montiert ist;
- eine Zelle zur Erfassung der Abweichung (25), montiert an einem zweiten Ende des Hebels (23) und konfigurier zum Erfassen einer Abweichung (Pos) des Hebels (23) von einer Gleichgewichtsposition;
- einen Zapfen (24), angeordnet unter dem Hebel (23), zwischen dem ersten und dem zweiten Ende, so dass die Verschiebung der Platte (22) durch die Schwerkraft zu einer Verschiebung mindestens eines Teils der Zelle zur Erfassung der Abweichung (25) führt;
- ein elektromagnetisches Gegengewicht (26), angeordnet unter dem Hebel (23) in Höhe des zweiten Endes; und
- einen Regler (27), verbunden mit der Zelle zur Erfassung der Abweichung (25) und dem elektromagnetischen Gegengewicht (26);
- der Regler (27) ist dabei zur Steuerung der elektrischen Energie (Im) des elektromagnetischen Gegengewichtes (26) in der Füllposition konfiguriert, um so einer von der Zelle zur Erfassung der Abweichung (25) erkannten Abweichung (Pos) entgegenzuwirken, das Gewicht (Mes) des Bechers (13) wird berechnet in Abhängigkeit von der elektrischen Energie (Im), die auf das elektromagnetische Gegengewicht (26) einwirkt, um einer Abweichung (Pos) dieses Hebels (23) entgegenzuwirken.

3. Verpackungsautomat nach Anspruch 2, ***bei dem*** diese Zelle zur Erfassung der Abweichung (25) umfasst
- eine Leuchtquelle (28) und eine Fotodiode (30), einander gegenüberliegend und bezogen auf ein Gehäuse (60) des Verpackungsautomaten (10a- 10b) fest montiert; und
- einen Verschluss, montiert auf dem zweiten Ende des Hebels (23), dieser Verschluss ist mit einer Öffnung (29) versehen, die zwischen der Leuchtquelle (28) und der Fotodiode (30) angeordnet ist, wenn sich der Hebel (23) in der Gleichgewichtsposition befindet.

4. Verpackungsautomat nach einem der Ansprüche 1 bis 3, ***bei dem*** diese Überwachungsvorrichtung (20) zur Steuerung der Pumpe (14a-14b) mit einem nichtlinearen Weg, mindestens zwei Füllgeschwindigkeiten umfasst, eine erste Geschwindigkeit bis zum Erreichen von mindestens 80% der verschriebenen Menge (Qua) und eine zweite Geschwindigkeit, die unter der ersten Geschwindigkeit liegt, bis zum Erreichen von 100% der verschriebenen Menge (Qua).

5. Verpackungsautomat nach einem der Ansprüche 1 bis 4, bei dem diese Befüllstation (PI) mehrere Vorratsbehälter (R2-R4) umfasst, die mehrere unterschiedliche Medikamente (P) enthalten, über jedem Vorratsbehälter (R2-R4) ist dabei eine Pumpe (14b) und ein Füllkopf (15b) montiert;
die Vorratsbehälter (R2-R4), die Pumpen (14b) und die zugehörigen erwähnten Füllköpfe (15a-15b) sind dabei auf einem in Rotation beweglichen Zylinder (55) montiert;
die Überwachungsvorrichtung (20) ist zur Steuerung der Verschiebung dieses Zylinders (55) konfiguriert, um den Füllkopf (15b) des Vorratsbehälters (R2-R4, der das verschriebene Medikament (P) enthält, über dem zu füllenden Becher (13) zu positionieren.

6. Verpackungsautomat nach Anspruch 5, ***bei dem*** jede Pumpe (14b) einen Kupplungsnippel (56) umfasst, bestimmt zum Zusammenwirken mit einer Aufnahmekupplung (57), montiert am Ende einer Motorachse (58), die Überwachungsvorrichtung (20) ist zur Steuerung der Kupplung des Motors (58) mit der Pumpe (14b) des Vorratsbehälters (R2-R4), der das verschriebene Medikament (P) enthält, bestimmt, wenn der Füllkopf (15b) des Vorratsbehälters (R2-R4) der das verschriebene Medikament (P) enthält, über dem zu füllenden Becher (13) positioniert ist.

7. Verpackungsautomat nach einem der Ansprüche 1 bis 6, ***bei dem*** dieser Verpackungsautomat (10a-10b) außerdem umfasst:
- eine Verdünnungsstation (P2);
- eine Station zum Aufsetzen eines Verschlusses (P3);
- eine Station zum Heißsiegeln eines Verschlusses (P4);
- eine Station zur Kennzeichnung des Verschlusses (P5);
- eine Station zur Kontrolle und Markierung des Bechers (P6); und
- eine Station zum Sortieren der konformen/ nicht konformen Becher (P7);
die Überwachungsvorrichtung (20) ist zur Steuerung der Verschiebung der Beförderungsvorrichtung (16a-16b) zwischen den verschiedenen Stationen (P1-P7) konfiguriert.

8. Verpackungsautomat nach einem der Ansprüche 1 bis 7, ***bei dem*** dieser Verpackungsautomat (10a-10b) auch eine Wartungsstation (P8) des mindestens einen Vorratsbehälters (R1-R4) des Medikamentes (P) umfasst, diese Wartungsstation (P8) umfasst dabei:
- ein RFID- Tag- Lesegerät (71), konfiguriert zum Lesen eines RFID- Tags, auf mindestens einem Vorratsbehälter (R1-R4) und ein RFID- Tag auf einem Austauschbehälter des Medikamentes (P), das im Vorratsbehälter (R1-R4) enthalten ist; und
- eine Mensch- Maschinen- Schnittstelle (72), die es einer Bedienperson ermöglicht, die Menge des nachgefüllten Medikamentes (P) im Vorratsbehälter (R1-R4) bei einer Füllphase des mindestens einen Vorratsbehälters (R1-R4) des Medikaments (P) anzugeben;
- die Überwachungsvorrichtung (20) ist dabei dazu konfiguriert, bei den Nachfüllphase die verbleibende Menge des Medikamentes (P) in dem mindestens einen Vorratsbehälter (RI-R4) des Medikamentes (P) mittels einer Waage zu schätzen, um die Bedienperson an der Mensch- Maschinen- Schnittstelle (72) zu informieren, wenn der mindestens eine Vorratsbehälter (R1-R4) des Medikamentes (P) eine Füllmenge aufweist, die unter einem Schwellenwert liegt.

9. Verpackungsverfahren für verschriebene Medikamente, umgesetzt von einem Verpackungsautomaten (10a-10b) nach Anspruch 7, wobei dieses Verfahren die folgenden Schritte umfasst:
- Verschiebung (100) der Beförderungsvorrichtung (16a-16b) unter eine Stapelsäule (80) mit Bechern (13), so dass ein Becher (13) in einer Öffnung (17) aufgenommen wird;
- Verschiebung (101) der Beförderungsvorrichtung (16a-16b) zwischen der Stapelsäule (80) und der Befüllstation (PI);
- Anheben (102) der Waage (21) in die Füllposition, so dass der angehobene Becher (13) nicht länger von der Beförderungsvorrichtung (16a-16b) gehalten wird;
- Verschiebung (103) des Zylinders (55), um den Füllkopf (15b) des Vorratsbehälters (R1-R4), der das verschriebene Medikament (P) enthält, über den zu füllenden Becher (13) zu bringen;
- Verkupplung des (104) Motors (58) mit der Pumpe (14b) des Vorratsbehälters (R1-R4), der das verschriebene Medikament (P) enthält;
- Füllen (105) des Bechers (13) entsprechend dem von der elektromagnetischen Kraftkompensationswaage (21) gemessenen Gewichts (Mes) bis das gemessene Gewicht (Mes) der verschriebenen Menge (Qua) entspricht; und
- Auskuppeln (106) des Motors (58) und der Pumpe (14b) und Absenken der Platte (22) in die Transportposition.

10. Verpackungsverfahren nach Anspruch 9, ***bei dem*** das Verfahren die folgenden Schritte umfasst:
- Verschieben (107) der Beförderungsvorrichtung (16a-16b) zwischen der Befüllstation (PI) und einer Verdünnungsstation (P2);
- Verdünnen (108) des verschriebenen Medikamentes (P) mit einer vorher festgelegten Menge;
- Verschieben (109) der Beförderungsvorrichtung (16a-16b) zwischen der Verdünnungsstation (P2) und einer Station zum Aufsetzen eines Verschlusses (P3);
- Aufsetzen (110) des Verschlusses;
- Verschieben (111) der Beförderungsvorrichtung (16a-16b) zwischen der Station zum Aufsetzen eines Verschlusses (P3) und einer Station zum Heißsiegeln des Verschlusses (P4);
- Verschweißen (112) des Verschlusses auf dem Becher (13);
- Verschieben (113) der Beförderungsvorrichtung (16a-16b) zwischen der Station zum Heißsiegeln des Verschlusses (P4) und einer Station zur Kennzeichnung des Verschlusses (P5);
- Markierung (114) auf dem Verschluss mindestens einer Information zum ausgegebenen Medikament (P), und der ausgegebenen Menge (Qua);
- Verschieben (115) der Beförderungsvorrichtung (16a-16b) zwischen der Station zur Kennzeichnung des Verschlusses (P5)) und einer Station zur Kontrolle und Markierung des Bechers (P6);
- Markierung (116) auf dem Becher mindestens einer Information zum Patienten (Des),
- Kontrolle (117) der auf dem Becher (13) und dem Verschluss angegebenen Informationen mit den Informationen in der Datenbank (12); und
- Verschieben (118) der Beförderungsvorrichtung (16a-16b) zwischen der Station zur Kontrolle und Markierung des Bechers (P6) und einer Station zum Sortieren der konformen/ nicht konformen Becher (P7).

## Claims

1. An automaton (10a-10b) for packaging prescriptions for a set of patients (Des), by means of a database (12) comprising a prescribed quantity (Qua) of a drug (P) associated with each patient (Des), said automaton (10a-10b) comprising:
- cups (13) intended to be dispensed to the various patients (Des) with the prescribed quantity (Qua) of drug (P);
- a conveyor (16a-16b) for moving the cups (13) and having through openings (17) for receiving the cups (13) in order to move them to a filling station (P1), including:
▪ at least one reservoir (R1-R4) of drug (P);
▪ a filling head (15a-15b) for dispensing the drug (P);
▪ a pump (14a-14b) connected between the reservoir (R1-R4) and the filling head (15a-15b); and
▪ a motor (58) operating the pump so as to dispense the drug (P) contained in the reservoir (R1-R4) at the filling head (15a-15b); and
- a monitoring member (20), connected to the database (12), configured to control the pump (14a-14b) so as to dispense the prescribed amount (Qua);
***characterized in that*** said filling station (P1) also comprises:
- a compensating electromagnetic force balance (21) comprising a plate (22), and an actuator for moving said balance (21) configured to move said balance (21) between two positions:
▪ a filling position in which the plate (22) lifts a cup (13) toward said dispensing head (15a-15b) so that the lifted cup (13) is no longer supported by the conveyor (16a-16b) and so that the balance (21) can measure the change in weight (Mes) of the lifted cup (13) during filling thereof; and
▪ a conveying position in which said plate (22) is not in contact with a cup (13) so that the conveyor (16a-16b) can move the cups (13);
- said monitoring member (20) being configured to control the motor (58) according to the measured weight (Mes) by said compensating electromagnetic force balance (21) until the measured weight (Mes) corresponds to the prescribed quantity (Qua).

2. The packaging automaton according to claim 1, wherein said compensating electromagnetic force balance (21) comprises:
- a lever (23) at a first end of which the plate (22) is mounted;
- a deflection detection cell (25) mounted on a second end of the lever (23) and configured to detect a deflection (Pos) of the lever (23) from an equilibrium position;
- a pivot (24) placed under the lever (23) between the first and the second end so that the movements of the plate (22) due to the gravity force cause a movement of at least a portion of the deflection detection cell (25);
- an electromagnetic counterweight (26) disposed under the lever (23) at the second end; and
- a regulator (27), connected to the deflection detection cell (25) and to the electromagnetic counterweight (26);
- the regulator (27) being configured to control, in the filling position, the electrical energy (Im) of the electromagnetic counterweight (26) so as to oppose a deflection (Pos) detected by the deflection detection cell (25), the weight (Mes) of the cup (13) being calculated in accordance with the electrical energy (Im) imposed on the electromagnetic counterweight (26) to oppose a deflection (Pos) of said lever (23).

3. The packaging automaton according to claim 2, wherein said deflection detection cell (25) comprises:
- a light source (28) and a photodiode (30), mounted facing each other and fixed with respect to a housing (60) of the packaging automaton (10a-10b); and
- a shutter mounted on the second end of the lever (23), said shutter being provided with an opening (29) arranged between the light source (28) and the photodiode (30) when the lever (23) is in the equilibrium position.

4. The packaging automaton according to any one of claims 1 to 3, wherein said monitoring member is configured to control the pump with a non-linear stroke comprising at least two filling speeds, a first speed until at least 80% of the prescribed quantity (Qua) is reached, and a second speed, lower than the first speed, until 100% of the prescribed quantity (Qua) is reached.

5. The packaging automaton according to any one of claims 1 to 4, wherein said filling station (P1) comprises a plurality of reservoirs (R2-R4) containing a plurality of distinct drugs (P), each reservoir (R2-R4) being surmounted by a pump (14b) and a filling head (15b);
the reservoirs (R2-R4), the pumps (14b) and said associated filling heads (15a-15b) being mounted on a rotatable barrel (55);
the monitoring member (20) being configured to control the movement of the barrel (55) in order to place the filling head (15b) of the reservoir (R2-R4) containing the prescribed drug (P) above the cup (13) to be filled.

6. The packaging automaton according to claim 5, wherein each pump (14b) includes a male coupling mechanism (56) for cooperating with a female coupling mechanism (57) mounted at the end of a shaft of the motor (58), the monitoring member (20) being configured to control the coupling of the motor (58) with the pump (14b) of the reservoir (R2-R4) containing the prescribed drug (P) when the filling head (15b) of the reservoir (R2-R4) containing the prescribed drug (P) is placed above the cup (13) to be filled.

7. The packaging automaton according to any one of claims 1 to 6, wherein said packaging automaton (10a-10b) also comprises:
- a dilution station (P2);
- a station (P3) for depositing a foil seal;
- a station (P4) for heat-sealing the foil seal;
- a station (P5) for marking the foil seal;
- a station (P6) for checking and marking the cup; and
- a station (P7) for sorting compliant/non-compliant cups;
the monitoring member (20) being configured to control the movement of the conveyor (16a-16b) between the different stations (P1-P7).

8. The packaging automaton according to any one of claims 1 to 7, wherein said packaging automaton (10a-10b) also comprises a maintenance station (P8) for the at least one reservoir (R1-R4) of drug (P), said maintenance station (P 8) comprising:
- an RFID tag reader (71) configured to read an RFID tag present on the at least one reservoir (R1-R4) and an RFID tag present on a replacement container of the drug (P) contained in the reservoir (R1-R4); and
- a man-machine interface (72) allowing an operator to indicate the quantity of drug (P) restocked in the reservoir (R1-R4) during a filling phase of the at least one reservoir (R1-R4) of drug (P);
- the monitoring member (20) being configured to estimate, during the filling phases, the remaining quantity of drug (P) in the at least one reservoir (R1-R4) of drug (P) by means of a balance in order to inform the operator interacting with the human-machine interface (72) when the at least one reservoir (R1-R4) of drug (P) has a filling quantity less than a threshold value.

9. Method for packaging prescriptions implemented by a packaging automaton (10a-10b) according to claim 7, said method comprising the following steps:
- moving (100) the conveyor (16a-16b) under a storage column (80) of cups (13) so that a cup (13) is received through an opening (17);
- moving (101) the conveyor (16a-16b) between the storage column (80) and the filling station (P1);
- lifting (102) the balance (21) in the filling position so that the lifted cup (13) is no longer supported by the conveyor (16a-16b);
- moving (103) the barrel (55) to place the filling head (15b) of the reservoir (R1-R4) containing the prescribed drug (P) above the cup (13) to be filled;
- coupling (104) the motor (58) with the pump (14b) of the reservoir (R1-R4) containing the prescribed drug (P);
- filling (105) the cup (13) according to the measured weight (Mes) by the compensating electromagnetic force balance (21) until the measured weight (Mes) corresponds to the prescribed quantity (Qua); and
- uncoupling (106) the motor (58) and the pump (14 b) and lowering the plate (22) into the conveying position.

10. The packaging method according to claim 9, wherein said method further comprises the following steps:
- moving (107) the conveyor (16a-16b) between the filling station (P1) and a dilution station (P2);
- diluting (108) the prescribed drug (P) with a predetermined amount;
- moving (109) the conveyor (16a-16b) between the dilution station (P2) and a station (P3) for depositing a foil seal;
- depositing (110) the foil seal;
- moving (111) the conveyor (16a-16b) between the station (P3) for depositing a foil seal and a station (P4) for heat-sealing the foil seal;
- welding (112) the foil seal to the cup (13);
- moving (113) the conveyor (16a-16b) between the station (P4) for heat-sealing the foil seal (P4) and a station (P5) for marking the foil seal;
- marking (114), on the foil seal; at least information relating to the drug (P) dispensed and the quantity (Qua) dispensed;
- moving (115) the conveyor (16a-16b) between the station (P5) for marking the foil seal and a station (P6) for checking and marking the cup;
- marking (116), on the cup (13); at least one information relating to the patient (Des);
- checking (117) the information marked on the cup (13) and the foil seal with the information contained in the database (12); and
- moving (118) the conveyor (16a-16b) between the station (P6) for checking and marking the cup and a station (P7) for sorting compliant/non-compliant cups.
